# EUROPEAN PATENT APPLICATION

(11) **EP 2 014 282 A2**
(43) Date of publication of application: **14.01.2009**
(21) Application number: 08011241.0
(22) Date of filing: 20.06.2008
(51) Int. Cl.: A61K 31/05, A61K 31/055, A61K 31/135, A61K 31/137, A61K 31/167, A61K 31/194, A61K 31/29, A61K 31/343, A61K 31/415, A61K 31/485, A61K 31/5415, A61K 31/616, A61K 45/06, A61P 25/24

(54) **Treatment of depression**

(30) Priority: 22.06.2007 GB 0712101; 07.09.2007 US 967760 P
(71) Applicant: E-Therapeutics plc, Newcastle Upon Tyne Tyne and Wear NE2 4LZ (GB)
(72) Inventor: Young, Malcolm Philip, Newcastle Upon Tyne Tyne & Wear, NE2 4LZ (GB); Yates, Catherine Mary, Newcastle Upon Tyne Tyne & Wear, NE2 4LZ (GB); Idowu, Olusola Clement, Newcastle Upon Tyne Tyne & Wear, NE2 4LZ (GB); Charlton, Julie Ann, Newcastle Upon Tyne Tyne & Wear, NE2 4LZ (GB)
(74) Representative: Gilholm, Stephen Philip

(57) **Abstract**

There is described a compound selected from the group consisting of tramadol, resveratrol, acetaminophen, xorphanol, cinfenoac, furcloprofen, bismuth subsalicylate, enofelast, triflusal, ketorfanol, indriline, furofenac, cizolirtine, dacemazine, demelverine, and fenethazine, and derivatives and/or combinations thereof, for the treatment or alleviation of depression.

There is also described a method of treating a patient suffering from depression.

## Description

### FIELD OF THE INVENTION

The present invention provides medicaments and methods for the treatment of depression.

### BACKGROUND

According to the World Health Organisation's latest study, depression ranks among the 10 leading causes of disability. The WHO study estimates that depression will become the second largest cause of the global health burden by 2020 (WHO 2001).

Already, more than 120 million people currently suffer from a depressive disorder for which they require treatment. Many people are afflicted by a depressive disorder at some point in their lives: the WHO estimates that 17% of all men and women will suffer from a depressive disorder during their life. Women suffer from depression more frequently than do men. It is estimated that 6% of all men and 9.5% of all women will suffer from a depressive disorder in any given year.

It is also estimated that many people who are in fact suffering from depression are not correctly diagnosed clinically as depressed. It is estimated that only about 40% of patients requiring treatment for depression in western countries and Japan were correctly diagnosed with depression in 2001 (Decision Resources, Psychiatric Disorders Study 1, November 2002). These estimates reflect a degree of uncertainty about the nature and mechanisms of depression. The term "depression" can refer to a very wide range of symptoms, from people who are able to hold down a job and function at a high level, to those who do not eat or drink, do not speak or move, and are candidates for emergency electro-convulsive therapy.

Almost all older accounts of the mechanisms of depression relate to abnormal brain chemistry, and explanations involve changes to the concentrations or patterns of release of neurotransmitters in central brain systems that mediate a patient's mood. In these explanations, the patient's behaviour is a result of abnormal function in these central brain systems controlling mood.

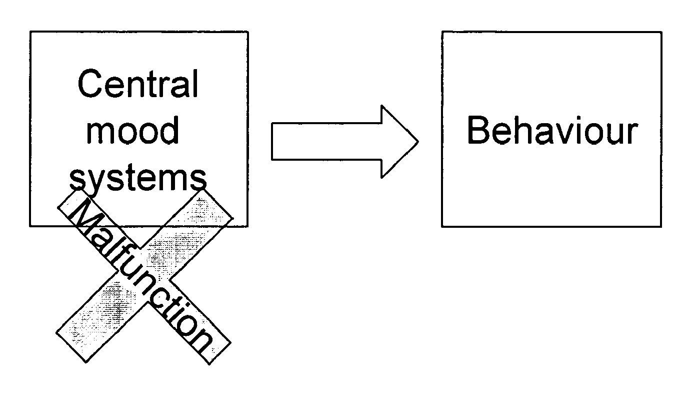

Treatment is similarly explained as an intervention on the neurotransmitter system in this malfunctioning central brain system controlling mood. 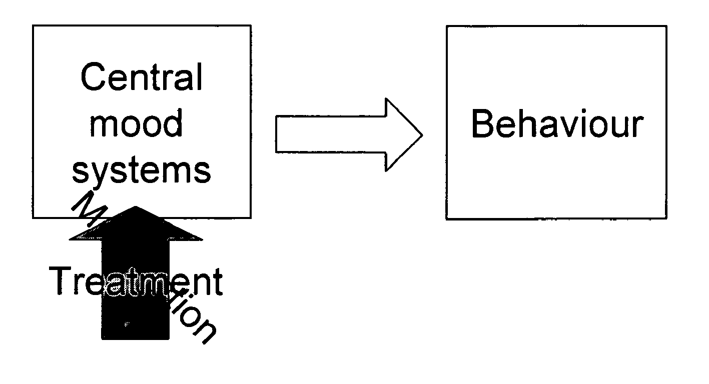 However, more recent accounts of depression relate to a more complex understanding of the systems and processes involved in mediating depression (e.g. Charlton B.G. Psychiatry and the human condition. Radcliffe Medical Press: Oxford, 2000). These accounts note the very close similarity between the behavioural characteristics of patients with a major depressive disorder and the behavioural characteristics of an evolved sickness behaviour, which was first described in animals. Both involve fatigue, somnolence, psychomotor retardation, impaired cognitive function, and demotivation with respect to normal drives and appetites. In the case of sickness behaviour, the evolved function of the behaviour is argued to be to derive a state in which the animal's energy is conserved, risks of further damage are minimised, and the immune system function is enhanced. Disorder of the systems involved in mediating this behaviour is then argued to be implicated in yielding depressive behaviour in people. In these modem accounts of depression, the underlying systems may be characterised in the following way:

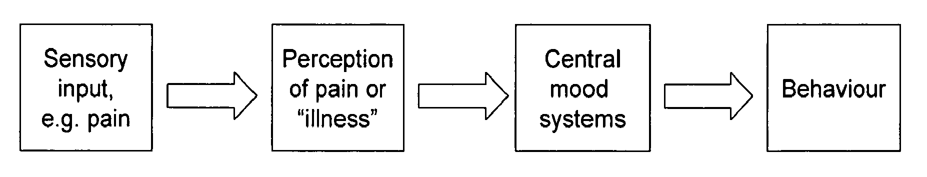

This set of systems can take on a number of different states. In a first example, no pain input is present, the system responsible for perception of pain correctly signals that no pain is present, the central mood systems are not affected, and normal behaviour is output. In a second example, pain is signalled in sensory input, the perceptual system correctly signals this pain, the central mood systems are depressed, and an appropriate energy-conserving and minimizing behaviour is output. In a third example, no pain is signalled in sensory input, but the perceptual system malfunctions and signals a pain condition to the central mood systems, which are then depressed, and an malfunction-derived depressed behaviour is output. In a fourth example, which mirrors the older models of depression, both sensory input and perception of pain are normal, but the central mood system malfunctions, and an inappropriate depressed behaviour is output. In a fifth example, sensory input is normal but both the system responsible for perception of pain and the central mood system malfunction, and an inappropriate depressed behaviour is output.

Treatment approaches follow from these permutations of possible function and malfunction in these systems. In the case that all systems are functioning normally, and no pain input is present, no treatment is necessary. In the case that a peripheral illness is generating pain input, which is then correctly interpreted by the other systems, the peripheral illness should be treated, if it can be. In the case that only the central mood system is disordered, a conventional antidepressant may be indicated. In all other cases, however, the appropriate treatment would involve simultaneously addressing depression with an analgesic to treat malfunction (or peripherally intractable pain inputs) of the first two elements of the system, and an antidepressant, to treat malfunction of the central mood systems. This is shown diagrammatically below:

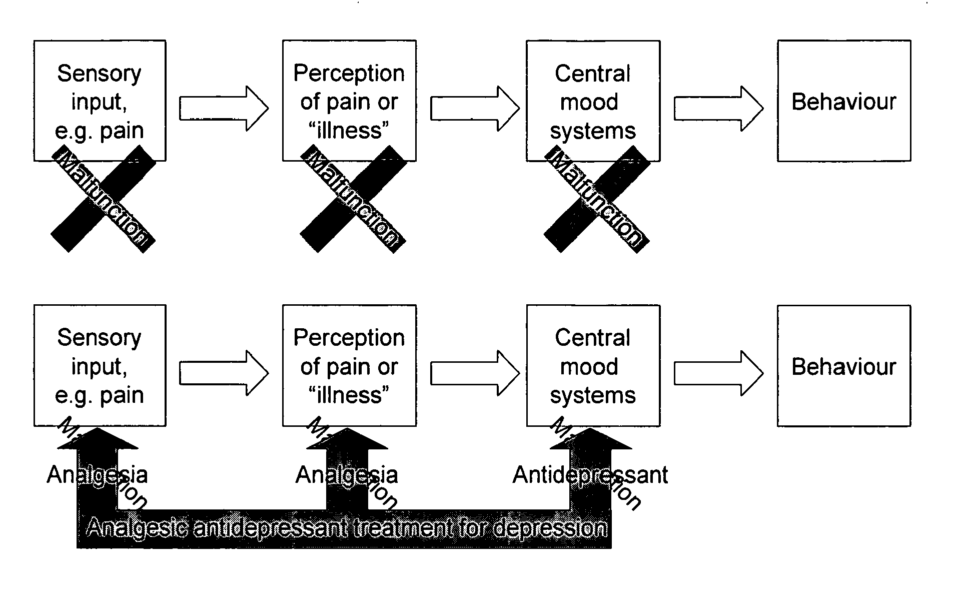

An objective of the present invention is to provide an effective treatment for depression, whose mode of action involves multiple interventions in the several sensory, perceptual and mood systems of the brain that are involved in mediating depression.

### SUMMARY OF THE INVENTION

We have now found a group of compounds that have the surprising effect of being efficacious in the treatment of depression through making multiple interventions on the several sensory, perceptual and mood systems of the brain that are involved in mediating depression.

Therefore, in accordance with a first aspect, the present invention provides the compounds selected from the group consisting of tramadol, resveratrol, acetaminophen, xorphanol, cinfenoac, furcloprofen, bismuth subsalicylate, enofelast, triflusal, ketorfanol, indriline, furofenac, cizolirtine, dacemazine, demelverine, and fenethazine and derivatives and/or combinations thereof, for the treatment or alleviation of depression.

We especially provide a compound as hereinbefore described for the treatment or alleviation of depression contributed to or caused by pain.

The compounds of group of the invention are each advantageous, *inter alia,* in that they are efficacious in the treatment or alleviation of pain whilst also treating depression. Thus, the compounds of the present invention are useful as analgesic antidepressants.

The compounds of the invention may each treat pain and depression separately, simultaneously or sequentially.

A preferred compound according to the invention is a compound selected from the group consisting of tramadol, resveratrol, dacemazine, demelverine, and fenethazine, and derivatives and/or combinations thereof.

Thus, in accordance with a further aspect of the invention we provide the use a compound selected from the group consisting of tramadol, resveratrol, acetaminophen, xorphanol, cinfenoac, furcloprofen, bismuth subsalicylate, enofelast, triflusal, ketorfanol, indriline, furofenac, cizolirtine, dacemazine, demelverine, and fenethazine and derivatives and/or combinations thereof, in the manufacture of a medicament for the treatment or alleviation of depression.

We further provide the use of a compound as hereinbefore described in the manufacture of a medicament for the treatment or alleviation of depression caused by or contributed to by pain.

These example chemicals derive the effect of being both analgesic and antidepressant by affecting specific proteins in the central nervous system. Research has shown that proteins including cyclooxygenase, oxidoreductase, histamine releasing proteins, indole-3-acetaldehyde oxidase, prolyl aminopeptidase, opioid delta receptor, opioid kappa receptor, opioid mu receptor, and corticotropin releasing factor are involved in mediating the suppression of pain signals. Research has shown that proteins including 2-haloacid dehalogenase, acetyl choline receptors and metabolic enzymes, adrenaline receptors and metabolic enzymes, dopamine receptors and metabolic enzymes, 5 hydroxytryptamine receptors and metabolic enzymes, monoamine receptors and metabolic enzymes, CC chemokine 2 receptor are involved in mediating central mood state and changes. These example chemicals derive the effect of being both analgesic and antidepressant by affecting the function one or more specific proteins that are involved in a mediating the suppression of pain signals, and one or more specific proteins that are involved in a mediating central mood state and changes.

The term "derivative" used herein shall include, *inter alia,* solvates. It may be convenient or desirable to prepare, purify, and/or handle a corresponding solvate of the compounds described herein, which may be used in any one of the uses/methods described. The term solvate is used herein to refer to a complex of solute, such as a compound or salt of the compound, and a solvent. If the solvent is water, the solvate may be termed a hydrate, for example a mono-hydrate, di-hydrate, tri-hydrate etc, depending on the number of water molecules present per molecule of substrate.

According to a further aspect of the invention we provide tramadol and/or a derivative or isomer thereof for the treatment or alleviation of depression, especially the treatment or alleviation of depression cause or contributed to by pain.

We also provide resveratrol and/or a derivative or isomer thereof for the treatment or alleviation of depression, especially the treatment or alleviation of depression cause or contributed to by pain.

We also provide acetaminophen and/or a derivative or isomer thereof for the treatment or alleviation of depression, especially the treatment or alleviation of depression cause or contributed to by pain.

We also provide xorphanol and/or a derivative or isomer thereof for the treatment or alleviation of depression, especially the treatment or alleviation of depression cause or contributed to by pain.

We also provide cinfenoac and/or a derivative or isomer thereof for the treatment or alleviation of depression, especially the treatment or alleviation of depression cause or contributed to by pain.

We also provide furcloprofen and/or a derivative or isomer thereof for the treatment or alleviation of depression, especially the treatment or alleviation of depression cause or contributed to by pain.

We also provide bismuth subsalicylate and/or a derivative or isomer thereof for the treatment or alleviation of depression, especially the treatment or alleviation of depression cause or contributed to by pain.

We also provide enofelast and/or a derivative or isomer thereof for the treatment or alleviation of depression, especially the treatment or alleviation of depression cause or contributed to by pain.

We also provide triflusal and/or a derivative or isomer thereof for the treatment or alleviation of depression, especially the treatment or alleviation of depression cause or contributed to by pain.

We also provide ketorfanol and/or a derivative or isomer thereof for the treatment or alleviation of depression, especially the treatment or alleviation of depression cause or contributed to by pain.

We also provide indriline and/or a derivative or isomer thereof for the treatment or alleviation of depression, especially the treatment or alleviation of depression cause or contributed to by pain.

We also provide furofenac and/or a derivative or isomer thereof for the treatment or alleviation of depression, especially the treatment or alleviation of depression cause or contributed to by pain.

We also provide cizolirtine and/or a derivative or isomer thereof for the treatment or alleviation of depression, especially the treatment or alleviation of depression cause or contributed to by pain.

We also provide dacemazine and/or a derivative or isomer thereof for the treatment or alleviation of depression, especially the treatment or alleviation of depression cause or contributed to by pain.

We also provide demelverine and/or a derivative or isomer thereof for the treatment or alleviation of depression, especially the treatment or alleviation of depression cause or contributed to by pain.

We also provide fenethazine and/or a derivative or isomer thereof for the treatment or alleviation of depression, especially the treatment or alleviation of depression cause or contributed to by pain.

Accordingly, and in one embodiment, the present invention provides a use of any one of tramadol, or resveratrol, or acetaminophen, or xorphanol, or cinfenoac, or furcloprofen, or bismuth subsalicylate, or enofelast, or triflusal, or ketorfanol, or indriline, or furofenac, or cizolirtine, or dacemazine, or demelverine, or fenethazine and/or derivatives thereof in the manufacture of a medicament for the treatment of depression.

Furthermore, in a second aspect, the present invention provides a method of treatment of a patient suffering from depression, said method comprising the administration of a therapeutically effective amount of one or more of the compounds selected from the group consisting of tramadol, resveratrol, acetaminophen, xorphanol, cinfenoac, furcloprofen, bismuth subsalicylate, enofelast, triflusal, ketorfanol, indriline, furofenac, cizolirtine, dacemazine, demelverine, and fenethazine, and derivatives and/or combinations thereof.

The method of the invention especially provides a method of treatment of a patient suffering from depression, contributed to or caused by pain.

Furthermore, the present invention encompasses a method of treating depression contributed to or caused by any type of pain or the misperception of any type of pain, said method comprising the step of administering a therapeutically effective amount of a compound as hereinbefore described and derivatives and/or a combination thereof.

According to a further aspect of the invention we provide a method as hereinbefore described wherein the compound is tramadol and/or a derivative or isomer thereof.

According to a further aspect of the invention we provide a method as hereinbefore described wherein the compound is resveratrol and/or a derivative or isomer thereof. According to a further aspect of the invention we provide a method as hereinbefore described wherein the compound is acetaminophen and/or a derivative or isomer thereof.

According to a further aspect of the invention we provide a method as hereinbefore described wherein the compound is xorphanol and/or a derivative or isomer thereof.

According to a further aspect of the invention we provide a method as hereinbefore described wherein the compound is cinfenoac and/or a derivative or isomer thereof.

According to a further aspect of the invention we provide a method as hereinbefore described wherein the compound is furcloprofen and/or a derivative or isomer thereof.

According to a further aspect of the invention we provide a method as hereinbefore described wherein the compound is bismuth subsalicylate and/or a derivative or isomer thereof.

According to a further aspect of the invention we provide a method as hereinbefore described wherein the compound is enofelast and/or a derivative or isomer thereof.

According to a further aspect of the invention we provide a method as hereinbefore described wherein the compound is triflusal and/or a derivative or isomer thereof. According to a further aspect of the invention we provide a method as hereinbefore described wherein the compound is ketorfanol and/or a derivative or isomer thereof.

According to a further aspect of the invention we provide a method as hereinbefore described wherein the compound is indriline and/or a derivative or isomer thereof.

According to a further aspect of the invention we provide a method as hereinbefore described wherein the compound is furofenac and/or a derivative or isomer thereof.

According to a further aspect of the invention we provide a method as hereinbefore described wherein the compound is cizolirtine and/or a derivative or isomer thereof.

According to a further aspect of the invention we provide a method as hereinbefore described wherein the compound is dacemazine and/or a derivative or isomer thereof.

According to a further aspect of the invention we provide a method as hereinbefore described wherein the compound is demelverine and/or a derivative or isomer thereof.

According to a further aspect of the invention we provide a method as hereinbefore described wherein the compound is fenethazine and/or a derivative or isomer thereof.

The compounds selected from the group tramadol, resveratrol, acetaminophen, xorphanol, cinfenoac, furcloprofen, bismuth subsalicylate, enofelast, triflusal, ketorfanol, indriline, furofenac, cizolirtine, dacemazine, demelverine, fenethazine and derivatives and/or combinations thereof are known *per se* and may be prepared using methods known to the person skilled in the art or may be obtained commercially.

A preferred group of compounds is the group consisting of tramadol, resveratrol, dacemazine, demelverine, and fenethazine, and derivatives and/or combinations thereof.

Advantageously, in the use and or method of the invention the compounds and/or derivatives and/or combinations thereof, may be administered orally, or intravenously.

Thus, compounds of the invention and/or derivatives and/or combinations thereof may be administered in combination with one or more other treatments known *per se.* For example, compounds of the invention and/or derivatives and/or combinations thereof may be administered in combination with one or more treatments for psychosis or other mental illness, where the combination of factors in an illness requires such combination treatment. Such combination therapies may comprise the separate, simultaneous or sequential administration of a compound of the invention with a treatment for psychosis or other mental illness, including, but not limited to, depression.

Examples of other medicaments known to be efficacious in the treatment or alleviation of depression include, but shall not be limited to, tricyclic medicaments, such as amitriptyline or omipramine; monoamine oxidase inhibitors, such as, phenelzine, isocarboxazide, tranylcypromine and moclobemide; and selective serotonin reuptake inhibitors, such as, fluoxetine, paroxetine, fluvoxamine, sertraline and escitalopram.

Pharmaceutical compositions of the compounds which form the subject matter of the invention are generally known. However, pharmaceutical compositions comprising combination therapies may be novel *per se* and may therefore form an additional aspect of the present invention.

According to a further aspect of the invention we provide a pharmaceutical composition comprising one or more of the compounds from the group consisting of tramadol, resveratrol, acetaminophen, xorphanol, cinfenoac, furcloprofen, bismuth subsalicylate, enofelast, triflusal, ketorfanol, indriline, furofenac, cizolirtine, dacemazine, demelverine, and fenethazine, and derivatives thereof, in admixture with a pharmaceutically acceptable adjuvant, diluent or carrier, for the treatment or alleviation of depression.

We further provide a pharmaceutical composition comprising one or more of the compounds of one or more of the compounds from the group consisting of tramadol, resveratrol, acetaminophen, xorphanol, cinfenoac, furcloprofen, bismuth subsalicylate, enofelast, triflusal, ketorfanol, indriline, furofenac, cizolirtine, dacemazine, demelverine, and fenethazine, and derivatives thereof, and a medicament for the treatment or alleviation of psychosis or other mental illness.

Thus, in the use, method and/or composition of the invention each of the compounds of group A may be put up as a tablet, capsule, dragee, suppository, suspension, solution, injection, e.g. intravenously, intramuscularly or intraperitoneally, implant, a topical, e.g. transdermal, preparation such as a gel, cream, ointment, aerosol or a polymer system, or an inhalation form, e.g. an aerosol or a powder formulation.

Compositions suitable for oral administration include tablets, capsules, dragees, liquid suspensions, solutions and syrups;
compositions suitable for topical administration to the skin include creams, e.g. oil-in-water emulsions, water-in-oil emulsions, ointments or gels;
examples of such adjuvants, diluents or carriers are:
   for tablets and dragees - fillers, e.g. lactose, starch, microcrystalline cellulose, talc and stearic acid; lubricants/glidants, e.g. magnesium stearate and colloidal silicon dioxide; disintegrants, e.g. sodium starch glycolate and sodium carboxymethylcellulose;
   for capsules - pregelatinised starch or lactose;
   for oral or injectable solutions or enemas - water, glycols, alcohols, glycerine, vegetable oils;
   for suppositories - natural or hardened oils or waxes.

It may be possible to administer a compound of the invention and/or derivatives and/or combinations thereof or any combined regime as described above, transdermally via, for example, a transdermal delivery device or a suitable vehicle or, e.g. in an ointment base, which may be incorporated into a patch for controlled delivery. Such devices are advantageous, as they may allow a prolonged period of treatment relative to, for example, an oral or intravenous medicament.

Examples of transdermal delivery devices may include, for example, a patch, dressing, bandage or plaster adapted to release a compound or substance through the skin of a patient. A person of skill in the art would be familiar with the materials and techniques which may be used to transdermally deliver a compound or substance and exemplary transdermal delivery devices are provided by GB2185187, US3249109, US3598122, US4144317, US4262003 and US4307717.

In a further embodiment, the methods and medicaments described herein may be used prophylactically as a means to prevent the development of depression, including depression contributed to or caused by any type of pain or the misperception of any type of pain. Medicaments and/or methods for prophylactic use may be administered or applied to any person at risk of developing depression, including depression contributed to or caused by any type of pain or the misperception of any type of pain.

For man the indicated total daily dosage may vary, but may be in the range of from 1mg to 3,000mg, preferably 5mg to 500, which may be administered in divided doses from 1 to 6 times a day or in sustained release form.

The invention will now be described by way of example only.

### Example 1

**A Single Centre, Open Labelled, Comparative, Parallel Pilot Study to Evaluate the Antidepressant Activity of Tramadol in the Treatment of Major Depressive Disorder (MDD).**

### PROTOCOL SYNOPSIS:

### INVESTIGATIONAL PRODUCT

Tramadol (Generic Name)

### DOSE/DOSAGE FORM/ROA

The investigational drug was administered in tablet form orally, with an initial dose of 200mg daily for 2 weeks and then maintenance at 200-400mg daily for a further 14 weeks.

### STUDY PATIENT POPULATION

Patients with MDD aged between 18 to 65 years old.

### OBJECTIVE

To evaluate the antidepressant activity of tramadol by comparing the change in Ham-D scores of patients with MDD when treated with tramadol as compared with amitriptyline.

### EFFICACY ENDPOINTS

Remission is defined as ≤ 7 on the 17 item Ham-D scale upon completion of week 8 of trial. Response is defined as ≥ 50% decrease from baseline depression on 17 item Ham-D score upon completion of week 8 of trial. Partial response is defined as <50% but ≥25% decrease from base line depression on 17 item Ham-D Scale. Recovery is defined as at least 8 weeks free from depression, as diagnosed according to DSM-IV-TR criteria.

### STUDY DESIGN

This was a comparative, single centre, parallel, open-label randomized study in which half of the study population will receive tramadol and the other half will receive amitriptyline.

### PRIMARY ENDPOINT

The primary endpoint is the treatment response of each study subject, as measured on the Ham-D 17-item scale (Hamilton MA). Study entry requires a Ham-D score of ≥15. To achieve remission, a cut-off score of ≤7 will be applied. Response will be defined as a ≥50% drop in score and a partial response will be defined as <50% but ≥25% drop in score. Recovery will be defined as at least 8 weeks free of major depression, as diagnosed according to DSM-IV-TR criteria.

### RESULTS

### Summary of initial clinical data from an ongoing study

In an open-label study comparing the treatment of Major Depressive Disorder with tramadol and amitriptyline, sixteen patients have been enrolled and five have completed the trial protocol. Of those five, three had received tramadol and two had received amitriptyline. In all cases, a clinically significant reduction in patient depression, as measured using the Hamilton-D rating scale, was observed. A further eight patients are displaying solid therapeutic responses and there have been three drop-outs. Information on therapeutic responses of the first 12 patients is summarised below.

| **Patient No.** | **Experimental drug** | **Ham-D change** |
|---|---|---|
| 1 | Tramadol | 23 to 0 |
| 3 | Tramadol | 23 to 0 |
| 4 | Amitriptyline | 22 to 0 |
| 5 | Tramadol | 26 to 4 (by penultimate visit) |
| 6 | Amitriptyline | 19 to 4 (by penultimate visit) |
| 8 | Tramadol | 26 to 4 (by penultimate visit) |
| 9 | Amitriptyline | 19 to 4 (by penultimate visit) |
| 10 | Tramadol | 26 to 0 (by penultimate visit) |
| 12 | Tramadol | 18 to 16 (by visit 4 of 7) |
| 2 | Amitriptyline | Dropped out after Visit 2 because of AE |
| 7 | Amitriptyline | Withdrawn because of non-compliance |

## Claims

1. A compound selected from the group consisting of tramadol, resveratrol, acetaminophen, xorphanol, cinfenoac, furcloprofen, bismuth subsalicylate, enofelast, triflusal, ketorfanol, indriline, furofenac, cizolirtine, dacemazine, demelverine, and fenethazine, and derivatives and/or combinations thereof, for the treatment or alleviation of depression.

2. A compound according to claim 1 wherein the compound is for the treatment or alleviation of depression contributed to or caused by pain.

3. A compound according to claim 1 wherein the compound is for the treatment or alleviation of pain and depression simultaneously, sequentially or separately.

4. A compound according to claim 1 wherein the compound is for prophylactic administration.

5. A compound according to claim 1 wherein the compound is selected from the group consisting of tramadol, resveratrol, dacemazine, demelverine, and fenethazine, and derivatives and/or combinations thereof.

6. A compound according to claim 1 wherein the compound is tramadol, and/or a derivative thereof.

7. A method of treatment or alleviation of a patient suffering from depression, said method comprising the administration of a therapeutically effective amount of one or more of the compounds selected from the group consisting of tramadol, resveratrol, acetaminophen, xorphanol, cinfenoac, furcloprofen, bismuth subsalicylate, enofelast, triflusal, ketorfanol, indriline, furofenac, cizolirtine, dacemazine, demelverine, and fenethazine and/or derivatives and/or combinations thereof.

8. A method according to claim 7 wherein the depression is contributed to or caused by pain.

9. A method according to claim 8 which comprises the treatment or alleviation of pain and depression simultaneously, sequentially or separately.

10. A method according to claim 7 wherein the method comprises prophylactic administration of a therapeutically effective amount of one or more of the compounds selected from the group consisting of tramadol, resveratrol, acetaminophen, xorphanol, cinfenoac, furcloprofen, bismuth subsalicylate, enofelast, triflusal, ketorfanol, indriline, furofenac, cizolirtine, dacemazine, demelverine, and fenethazine and/or derivatives and/or combinations thereof.

11. The method according to claim 7 wherein the compound is selected from the group consisting of tramadol, resveratrol, dacemazine, demelverine, and fenethazine, and derivatives and/or combinations thereof.

12. The method according to claim wherein the compound is tramadol and/or a derivative or isomer thereof.

13. A pharmaceutical composition comprising one or more of the compounds from the group consisting of tramadol, resveratrol, acetaminophen, xorphanol, cinfenoac, furcloprofen, bismuth subsalicylate, enofelast, triflusal, ketorfanol, indriline, furofenac, cizolirtine, dacemazine, demelverine, and fenethazine, and derivatives thereof, in admixture with a pharmaceutically acceptable adjuvant, diluent or carrier, for the treatment or alleviation of depression.

14. A pharmaceutical composition comprising one or more of the compounds of one or more of the compounds from the group consisting of tramadol, resveratrol, acetaminophen, xorphanol, cinfenoac, furcloprofen, bismuth subsalicylate, enofelast, triflusal, ketorfanol, indriline, furofenac, cizolirtine, dacemazine, demelverine, and fenethazine, and derivatives thereof, and a medicament for the treatment or alleviation of psychosis or other mental illness.

15. A pharmaceutical composition according to claim 14 wherein the medicament is selected from one or more of a tricyclic medicament, a monoamine oxidase inhibitor and a selective serotonin reuptake inhibitor.
